# EUROPEAN PATENT APPLICATION

(11) **EP 1 911 471 A1**
(43) Date of publication of application: **16.04.2008**
(21) Application number: 06766822.8
(22) Date of filing: 16.06.2006
(51) Int. Cl.: A61L 31/00, A61K 31/717, A61K 31/728, A61K 31/765, A61K 38/43, A61K 45/00, A61L 27/00, A61P 27/02, A61P 27/12, A61P 41/00, A61P 43/00

(54) **AGENT FOR VISUALIZING VITREOUS BODY**

(30) Priority: 22.06.2005 JP 2005182412
(71) Applicant: Kyoto University, Kyoto-shi, Kyoto 606-8501 (JP)
(72) Inventor: YOSHIMURA, Nagahisa, konoecho, Sakyo-ku,Kyoto-shi, Kyoto 6068501 (JP); MUSASHI, Kunihiro, konoecho, Sakyo-ku, Kyoto-shi, Kyoto 6068501 (JP); TABATA, Yasuhiko, Shogo-in, Sakyo-ku, Kyoto-shi, Kyoto 6068501 (JP)
(74) Representative: HOFFMANN EITLE
(86) International application number: PCT/JP2006/312134
(87) International publication number: WO 2006/137341

(57) **Abstract**

A vitreous body visualization agent which is to be injected into the eye to visualize the vitreous body by a safe and sure method. The vitreous body visualization agent is prepared by using a polymeric substance having positively or negatively charged functional groups and adding a hardly water-soluble and non-steroidal colored substance, in which the polymeric substance is to be suspended, thereto. Due to having a non-steroidal nature, it can be safely injected into the eye, and the colored substance, in which the polymeric substance is to be suspended, makes the vitreous body visually recognizable.

## Description

### TECHNICAL FIELD

The present invention relates to a vitreous body visualization agent.

### BACKGROUND ART

Traditionally, as one step during vitreous body surgery, resection of a vitreous body is performed. In resecting the vitreous body, it is important to completely remove the vitreous body from the retina. However, because the vitreous body itself is transparent, complete resection is difficult as is.

By contrast, it is known that when Kenacort (Registered Trademark), which has been developed and sold as a steroid anti-inflammatory agent for systemic illness, is injected into the vitreous body, the vitreous body is secondarily visualized (see non-patent document 1). When Kenacort (Registered Trademark) is used, it is possible to easily and reliably perform resection as the vitreous body can be clearly seen. By doing so, it is possible, for example, to create posterior vitreous body exfoliation in an extremely short period of time. Also, because the possibility of needlessly manipulating a vitreous body cutter decreases markedly, it is possible to avoid creating an iatrogenic hiatus in the surrounding part. Non-patent document 1: Gekkan Ganka June, 2001 issue (Vol. 43, No. 6), pp. 817-821 Surgical procedure "Vitreous body surgery visualizing a vitreous body by triamcinolone" by Taiji Sakamoto

### DISCLOSURE OF INVENTION

### OBJECT OF THE INVENTION

However, triamcinolone acetonide included in the above-described Kenacort (Registered Trademark) is a water-insoluble steroid agent that is known to cause, at a minimum, increased intraocular pressure, and has the possibility to cause major side-effects such as glaucoma and cataracts. Consequently, there are problems with using it only for the reason that it can be used to visualize the vitreous body. Steroid agents have the possibility of fundamentally aggravating infection, and it is necessary to proceed with further caution when using it in cases involving infections.

Therefore, the appearance of a substance that would enable visualization of the vitreous body without causing these side-effects is sought.

The present invention was made in view of the above-described problems in the conventional art, and the object of the invention is to provide a vitreous body visualization agent that is very safe.

### MEANS FOR SOLVING THE OBJECT

In order to achieve the above-described object, according to one aspect of the present invention,
a vitreous body visualization agent, which is injected intraocularly in order to visualize a vitreous body, comprises:
a polymeric substance having a functional group having either a positive or negative electrical charge; and
a colored substance which suspends the polymeric substance and which has poor water solubility and is non-steroidal.

The functional group may be a carboxyl group or an amino group.

The polymeric substance may be sodium carboxylcellulose.

The polymeric substance may be sodium hyaluronate.

The vitreous body visualization agent may further include an enzyme, and enzymatically melts the vitreous body by acting on a retinal vitreous body boundary surface.

The colored substance may be a polylactic acid or polylactic acid particles.

The colored substance may be a ceramic bone flap.

### EFFECT OF THE INVENTION

The present invention provides a vitreous body visualization agent which has higher safety.

### BRIEF DESCRIPTION OF DRAWINGS

FIG. 1 shows experimental results obtained from investigating adhesive properties towards a vitreous body made of polymeric substance.
FIG. 2 shows the state of having intraocularly injected a vitreous body visualization agent according to the present invention.
FIG. 3 shows the state of intraocularly injecting the vitreous body visualization agent, further performing exfoliation of artificial posterior vitreous body, and performing resection of surrounding vitreous body.

### BEST MODE FOR CARRYING OUT THE INVENTION

Preferred embodiments of the present invention will be described exemplarily in detail below. However, the substances described in these embodiments are merely exemplary, and are not meant to restrict the scope of the inventions to simply these substances.

### (First Embodiment)

### <Structure of the vitreous body visualization agent>

In visualizing a vitreous body, the minimum necessary factors are adhesiveness towards the vitreous body as well as being colored. In the vitreous body visualization agent according to the present embodiment, mixing of two substances, each fulfilling one of the necessary factors respectively, results in visualization being made possible.

Specifically, adhesiveness towards the vitreous body is realized by a polymeric substance having a functional group having either a positive or negative electrical charge. Coloredness is realized by a colored substance which suspends the polymeric substance and which has poor water solubility and is non-steroidal.

As a polymeric substance which will serve as a base agent for the vitreous body visualization agent, it is possible to select any of the polymeric substances described below.
(1) Carboxymethylcellulose Na

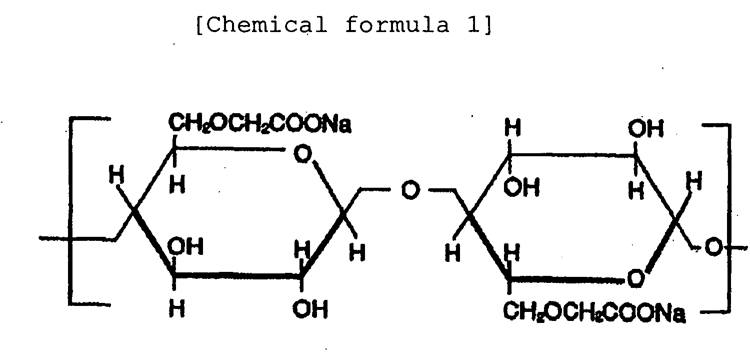

(2) Hydroxyethyl cellulose

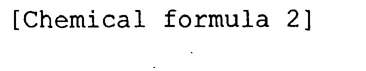

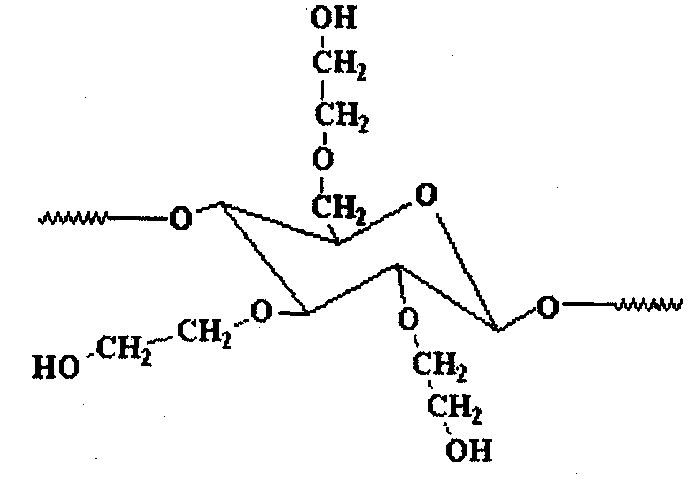

(3) Hyaluronic acid

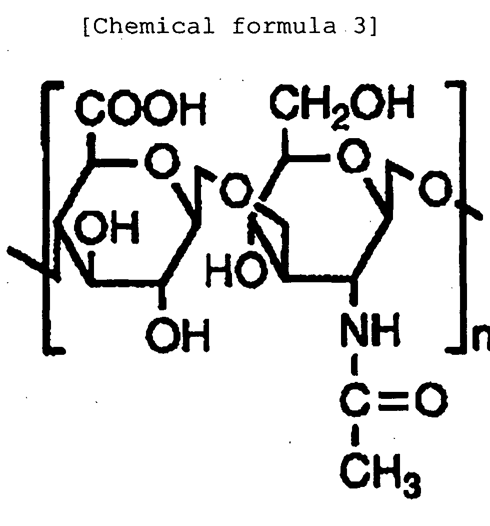

(4) Gelatin (R1 and R2 are side chains. Amino acids are connected in a straight chain.)

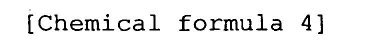

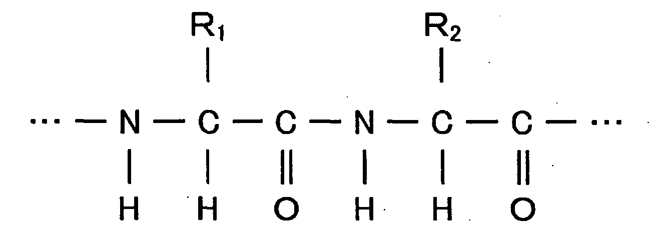

As described above, hyaluronic acid has many carboxyl groups. In addition, because gelatin is formed from polymerized amino acids, it contains a variety of functional groups. Among these, functional groups having electrical charge include amino groups (-NH2) and carboxyl groups (-COOH). An amino group has a positive electrical charge, and a carboxyl group has a negative electrical charge. Gelatin is charged either positively or negatively. By manipulating the functional groups, it is possible to increase the number of amino groups (-NH2) that are charged positively, or to increase the number of carboxyl groups (-COOH) that are charged negatively.
Experiments were carried out with gelatin in which the amount of electrical charge was changed in four stages.
Measurements were quantitatively made on how absorption of vitreous body and polymer are influenced by a gradient of electrical charge of the side chains. Bound to each gelatin is a fluorescent dye.
The procedures and results of the experiment are described below.

### (Generation of a polymer labeled with fluorescent dye)

Four types of gelatins (A-D) were prepared in which the functional groups of the side chains of the gelatins were modified and the isoelectric points varied in four stages. The preparation was made so that the FITC introduction ratio of each of the gelatins is the same.

### (Dry fixing of the vitreous body)

Vitreous body (jellified) isolated from pig eyes is agitated and pulverized (fluidized) using a tissue lyser. Vitreous body fluid 1ml is dried after placing in a Petri dish.

### (Contact between a polymer and the dried vitreous body)

Gelatin aqueous solutions (A-D) labeled with FITC are each injected into a different Petri dish, and 30 seconds are allowed to pass. After withdrawing gelatin aqueous solutions by suction and washing with ultrapure water, drying is performed. Excess gelatin is removed by this manipulation, and only gelatin adhering to the vitreous body remains.

### (Decomposition of vitreous body and measurement of fluorescence)

Proteinase K was used to enzymatically decompose the vitreous body. Then, the fluorescence intensity of the obtained sample was determined and the amount of FITC adhered to the vitreous body was found. In this way, the degree of adherence of the four types of gelatins to vitreous body was quantitatively compared.

### (Results)

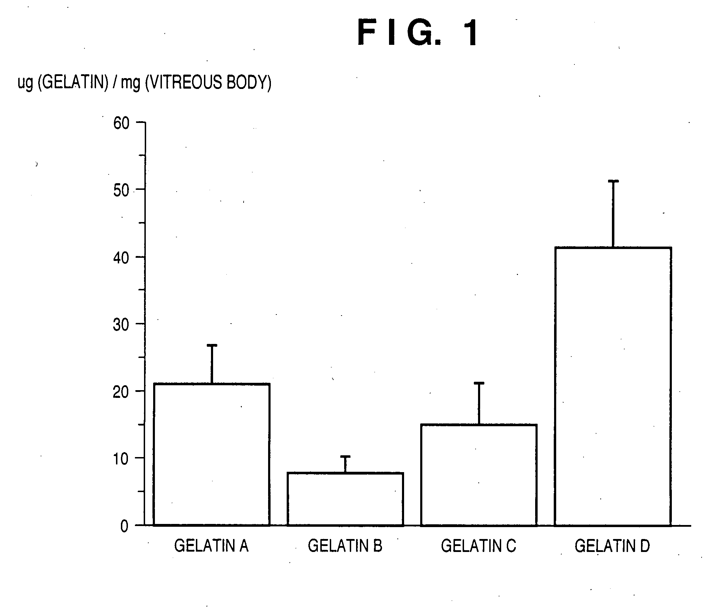

FIG. 1 shows the results of carrying out experiments. Gelatin A contains the largest number of carboxyl groups (-COOH) and gelatin D contains the largest number of amino groups (-NH2). B and C have intermediate numbers. Differences between A and B, A and D, B and D, and C and D are significant (p<0.05). It can be concluded that, compared to gelatins B and C, which are close to neutral in electrical charge, gelatin D, which is strongly positively charged, or gelatin A, which is strongly negatively charged, are more strongly adherent to the vitreous body. This proved that electrical charge plays an important role in the absorption of the vitreous body and the polymer.

In order to strongly maintain the degree of visualization of the vitreous body, it is necessary to strengthen the absorption of the polymer substance, which forms the base for the vitreous body visualization agent, with the vitreous body. It can be said from the above-described experimental results that it is necessary to choose, as a solvent, an aqueous solution of polymer substance having positive or negative electrical charge.

Carboxymethyl cellulose includes, as a functional group, the methyl carboxyl group (CH2-COOH). By contrast, there is a polymer aqueous solution which does not visualize a vitreous body, but the functional groups of this polymer include hydroxyethyl groups (CH2-CH2-OH). This functional group hardly has electrical charge.

From the above, it is possible to determine that a polymer substance having functional groups such as carboxyl groups (-COOH), methyl carboxyl groups (CH2-COOH), and amino groups (-NH2), each of the groups of which have either positive or negative electrical charges, adheres to the surface of the vitreous body and may be the base substance of the vitreous body visualization agent, but polymer substance having only functional groups such as hydroxyethyl groups (CH2-CH2-OH) which do not have electrical charge may not serve as the base substance of the vitreous body visualization agent.
The molecular weight of the polymer substance having adhesiveness towards a vitreous body is preferably 1,000 or more but 1,000,000 or below. This is because if the molecular weight is less than 1,000, the polymer substance would mix into the vitreous body, and does not diffuse if larger than 1,000,000. Specifically, for effective adhesion, the molecular weight of polymer substance is preferably several tens of thousands.

Because it is possible, by using the above-described polymer substance, to coat the vitreous body boundary surface (surface facing the operator), the undulation of the surface of the vitreous body itself during vitreous body surgery can be visualized by mixing a colored substance to the polymer substance.

As the colored substance, a substance with poor water solubility and which is non-steroidal is used. In this way, it is possible to prevent this substance from melting into the vitreous body and enables the prevention of the serious side-effects of steroid agents.

As the colored substance, a ceramic bone flap (β-TCP), polylactic acid particles, or polylactic acid, can be used. These are all white-colored powders, and their features are given below.

### (1) β-TCP

β- tricalcium phosphate. A substance which is used in bone flaps, and which is water insoluble and does not have bioactivity.

### (2) Polylactic acid

A polymer substance which is made of monomers of lactic acid <HC-(CH3)(OH)(COOH)> and which is a polymer substance polymerized repeatedly by ester linkage. Glycine <H2C- (OH) (COOH) > may also be added to the monomers. Polylactic acid is slowly hydrolytically cleaved in vivo, and is cleaved to carbon dioxide and water.

### (3) Polylactic acid particles

A processed substance in which straight-chain (2) is entangled in a fuzzball shape and is made into a spherical shape. It is possible to create polylactic acid particles of a diameter of several nanometers to several hundred micrometers.
The particle diameter of the coloring matter is preferably between about 100 nanometers and about 10 micrometers, and it is effective to make the weight concentration 1-10% of the total solution.
It is cautioned that, during mixing, the colored substance and the polymer substance be mixed well and agitated.

### <The effect of the vitreous body visualization agent on vitreous body resection surgery>

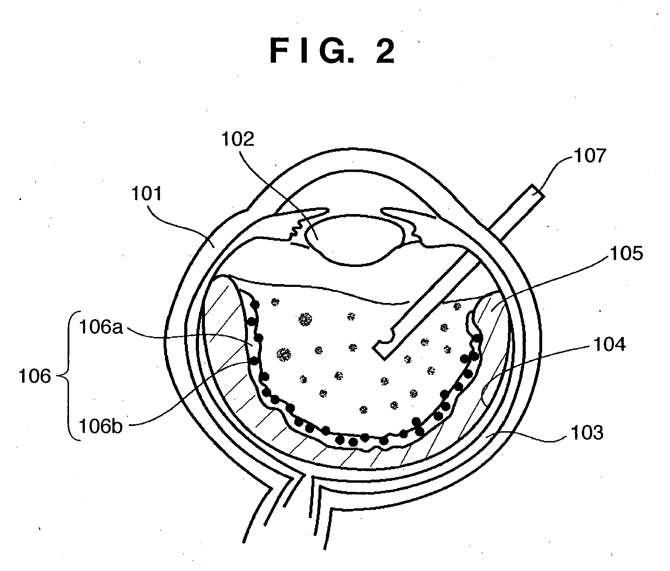

Resection of the vitreous body first begins from exfoliating the adhesive attachment between the retina and the vitreous body (artificial posterior vitreous body exfoliation). Injection of the vitreous body visualization agent is performed before this artificial rear vitreous body exfoliation. It is basically sufficient to inject the vitreous body visualization agent once. At this point, the interior of the eye becomes the state shown in FIG. 2. FIG. 2 is a cross-sectional view of an eye ball 101 directed upwards. Here, reference numeral 102 is a lens, 103 is a choroid, 104 is a retina, and 105 is a vitreous body. Generally, the vitreous body fills the space known as the vitreous body cavity below the lens 102. However, FIG. 2 shows a state in which more than half of the vitreous body has been removed by an instrument 107.
The vitreous body 105 is structured so that it is originally attached firmly to the retinal surface, but because the retina 104 and the vitreous body 105 are transparent, sufficient experience and intuition is required to separate them as is. At the same time, if the vitreous body visualization agent 106 is injected intraocularly, a polymer substance 106a spreads to the boundary of the vitreous body 105, attaches to the vitreous body 105, and a colored substance 106b enables the position and shape of the boundary of the vitreous body 105 to be visualized, as shown in FIG. 2. The positional relationship of the retinal surface and the vitreous body boundary is thus known, and it is possible to perform an artificial posterior vitreous body exfoliation in a very safe manner. Once this operation is complete, resection of the vitreous body 105 is begun in earnest.

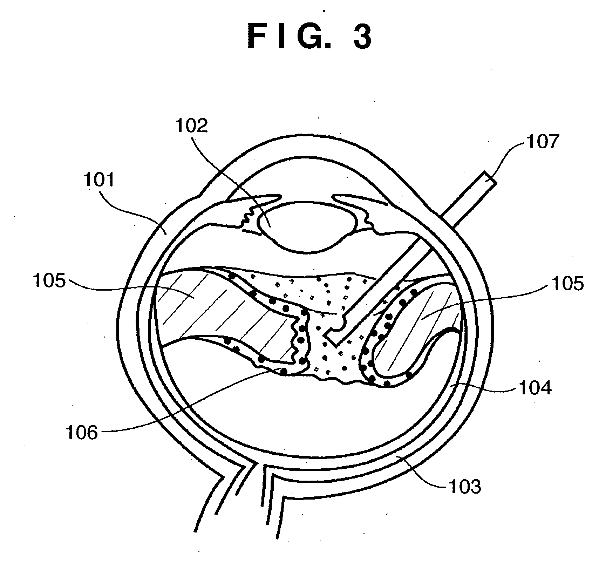

Once the adhesion between the retina 104 and the vitreous body 105 is removed, the state in which only the fundus (ring-shaped inner wall) of the vitreous body 105 and the retina 104 is reached, and, further, when part of the vitreous body 105 is removed so that holes are made near the center of the vitreous body 105, as shown in FIG. 3, part of the vitreous body visualization agent 106 is sterically attached to the back (the region that was originally attached to the retina) of the vitreous body 105.
In this state, resection of the surrounding vitreous body is performed. The degree of resection differs depending on the illness, but in order for the vitreous body visualization agent 106 to attach so as to cover the entire vitreous body, even in the case of nearly completely resecting the vitreous body to the vitreous body basal region, it is possible to reliably perform resection of the vitreous body while clearly confirming the degree of that resection.

## Claims

1. A vitreous body visualization agent which is injected intraocularly in order to visualize a vitreous body, comprising:
a polymeric substance having a functional group having either a positive or negative electrical charge; and
a colored substance which suspends the polymeric substance and which has poor water solubility and is non-steroidal.

2. The vitreous body visualization agent according to claim 1, wherein the functional group is a carboxyl group or an amino group.

3. The vitreous body visualization agent according to claim 1, wherein the polymeric substance is sodium carboxycellulose.

4. The vitreous body visualization agent according to claim 1, wherein the polymeric substance is sodium hyaluronate.

5. The vitreous body visualization agent according to claim 1, wherein the vitreous body visualization agent further includes an enzyme, and enzymatically melts the vitreous body by acting on a boundary surface between a retina and the vitreous body.

6. The vitreous body visualization agent according to claim 1, wherein the colored substance is a polylactic acid or polylactic acid particle.

7. The vitreous body visualization agent according to claim 1, wherein the colored substance is a ceramic bone flap.
